# EUROPÄISCHE PATENTSCHRIFT

(11) **EP 0 307 730 B1**
(45) Veröffentlichungstag und Bekanntmachung des Hinweises auf die Patenterteilung: **21.07.1993**
(21) Anmeldenummer: 88114309.3
(22) Anmeldetag: 02.09.1988
(51) Int. Cl.: C12N 15/61, C12N 9/90, C12P 19/02

(54) **Expression von Mutarotase**
Expression of mutarotase
Expression de mutarotase

(30) Priorität: 12.09.1987 DE 3730712
(43) Veröffentlichungstag der Anmeldung: 22.03.1989
(73) Patentinhaber: MERCK PATENT GmbH, D-64271 Darmstadt (DE)
(72) Erfinder: Hillen, Wolfgang, Prof. Dr., D-8520 Erlangen (DE); Schmucker, Robert, Dr., D-8523 Baiersdorf (DE); Gülland, Ulrike, D-8520 Erlangen (DE)

(56) Entgegenhaltungen:
- EP-A- 0 214 548
- GENE, Band 31, 1984, pages 103-108, Elsevier Science Publishers; B. UNGER et al.: "Nucleotide sequence of the gene, protein purification and characterization of the pSC101-encoded tetracycline resistance-gene-repressor"
- NUCLEIC ACIDS RESEARCH, Band 14, Nr. 10, 1986, pages 4309-4323, IRL Press Ltd, Oxford, GB; C. GATZ et al.: "Acinetobacter calcoaceticus encoded mutarotase: nucleotide sequence analysis of the gene and characterization of its secretion in Escherichia coli"

## Beschreibung

Die Erfindung betrifft die Bereitstellung rekombinanter DNA-Sequenzen und transformierte Wirtsorganismen, die für ein Polypeptid mit der biologischen Aktivität von Mutarotase codieren.

Außerdem betrifft die Erfindung Klonierungs- und Expressions-Vektoren, zur Verwendung bei der Herstellung eines Polypeptids mit der biologischen Aktivität von Mutarotase sowie mit solchen Vektoren transformierte Wirtsorganismen, beispielsweise Bakterien, Hefen, andere Pilze, tierische oder menschliche Zellen.

Schließlich betrifft die Erfindung die Verwendung des genannten Polypeptids mit der biologischen Aktivität des Enzyms Mutarotase bei der Beschleunigung enzymatischer Nachweisreaktionen oder Umsetzungen von Aldosen.

Mutarotase (Aldose-l-epimerase, EC 5.1.3.3) beschleunigt bekanntlich die Gleichgewichtseinstellung zwischen den α- und β-Anomeren von Aldohexosen, z.B. zwischen α-und β-Glucose oder α- und β-Galactose. Die Hauptanwendung des Enzyms liegt in der analytischen Biochemie bei der Beschleunigung enzymatischer Nachweisreaktionen für Aldosen mit Hilfe von für die α- oder β-Form spezifischen Enzymen, bei denen die Gleichgewichtseinstellung zwischen beiden Anomeren der geschwindigkeitsbestimmende Schritt ist, z.B. bei Bestimmungsmethoden mit Glucosedehydrogenase, Glucoseoxidase oder Galactosedehydrogenase.

Die Bestimmung der Mutarotase-Aktivität erfolgt in an sich bekannter Weise durch Messung der Reaktionsbeschleunigung des Glucoseumsatzes mit z.B. Glucosedehydrogenase. Dieses Verfahren zählt zu den Standardmethoden beispielsweise in der klinischen Chemie. Dabei läßt man Mutarotase, Glucosedehydrogenase und NAD mit einer frisch angesetzten Lösung von α-Glucose reagieren und bestimmt das gebildete NADH₂ durch Extinktionsmessung bei 366 nm. Die Messung der Beschleunigung der NADH₂-Bildung durch Mutarotase im Verhältnis zu einem Vergleichswert gestattet die Berechnung der Mutarotase-Aktivität der eingesetzten Lösung.

Mutarotase ist in der Natur weit verbreitet, sie kommt in verschiedenen Mikroorganismen (Bakterien, Hefen, Fadenpilzen), in Pflanzen und in tierischen Geweben vor.

Nennenswerte Enzymgehalte, die eine Isolierung von Mutarotase im technischen Maßstab ermöglichen, wurden bisher nur in Nieren von Säugetieren (Rind, Schwein) gefunden; alle bekannten Handelsprodukte werden aus Nieren hergestellt. Die Aufarbeitungsverfahren sind hierbei in der Regel recht aufwendig und man benötigt große Mengen Ausgangsmaterial. In Biochim. Biophys. Acta 662, 285 (1981) wird erstmals ein Verfahren zur mikrobiologischen Herstellung von Mutarotase aus Stämmen von Aspergillus niger beschrieben. Danach wurde aus dem besten Stamm eine Mutarotase-Aktivität von 4,4 U/l Kulturbrühe erzielt.

Die Ausbeute an Mutarotase ist jedoch nach diesem Verfahren verhältnismäßig niedrig. Außerdem sind die Eigenschaften des Enzyms aus Aspergillus niger, insbesondere bei Einsatz zu enzymatischen Bestimmungen, ungünstig. Aus der DE-OS-3531360 ist bekannt, daß in optimierten Stämmen von Acinetobacter calcoaceticus Mutarotase-Aktivitäten nach klassischen mikrobiellen Verfahren von 50-100 U/l Kulturbrühe erreicht werden können. Vergleichsweise hierzu wird in derselben Offenlegungsschrift ein gentechnologisches Verfahren beschrieben, bei dem in einem transformierten E. coli-Wirt ein Polypeptid mit der biologischen Aktivität von Mutarotase exprimiert wird, welches von einer DNA codiert wird, die vorzugsweise aus dem Genom von Acinetobacter calcoaceticus stammt. Dabei werden Mutarotase-Aktivitäten von durchschnittlich etwa 5000 U/l Kulturbrühe erzielt. Auch diese gegenüber den klassischen Verfahren bereits stark verbesserte Aktivitäten bzw. Ausbeuten an Mutarotase erscheinen aber immer noch nicht ausreichend.

Der Erfindung liegt somit die Aufgabe zugrunde, DNA-Sequenzen und Polypeptide bereitzustellen, welche die Herstellung von Mutarotase innerhalb einer gentechnologischen Verfahrens in deutlich höheren Ausbeuten als bisher. und mit guten Enzymeigenschaften ermöglichen.

Überraschend wurde gefunden, daß durch Entfernen der ersten 60 Nukleotide der in der DE-OS-3531360 angegebenen, für ein Polypeptid mit der biologischen Aktivität von Mutarotase codierenden DNA-Sequenz, geringfügigen, weiter unten näher spezifizierten Änderungen innerhalb der nachfolgenden 21 Nukleotide des Strukturgens und Fusionierung dieses neuen Strukturgens mit dem Genstart des Tetracyclin-Repressors sowie mit einer effektiven Promotor-Sequenz, die beide aus dem gleichen Mikroorganismus stammen, in dem die Expression des Enzyms durchgeführt wird, ein neues rekombinantes DNA-Molekül erhalten wird, das in dem transformierten Wirtsorganismus die Expression stabiler Mutarotase bewirkt, wobei die erreichbaren Enzymaktivitäten um den Faktor 10 bis 50 höher liegen als bei der Mutarotase-Gewinnung des gentechnischen Verfahrens der DE-OS-3531360. Die Eigenschaften der nach dem erfindungsgemäßen Verfahren hergestellten Mutarotase gleichen den vorteilhaften Eigenschaften der in der DE-OS-3531360 gewonnenen Enzyms.

Gegenstand der Erfindung ist somit eine rekombinante DNA-Sequenz, codierend für ein Polypeptid mit der biologischen Aktivität von Mutarotase, dadurch gekennzeichnet, daß sie das Strukturgen aus dem Genom eines Mutarotase bildenden Mikroorganismus, den Genstart des Tetracyclin-Repressors und eine Promotor-Sequenz enthält, wobei der Sequenzbereich des Tetracyclin-Repressors und die Promotor-Sequenz aus dem Mikroorganismus stammen, in dem die Expression des Strukturgens durchgeführt wird.

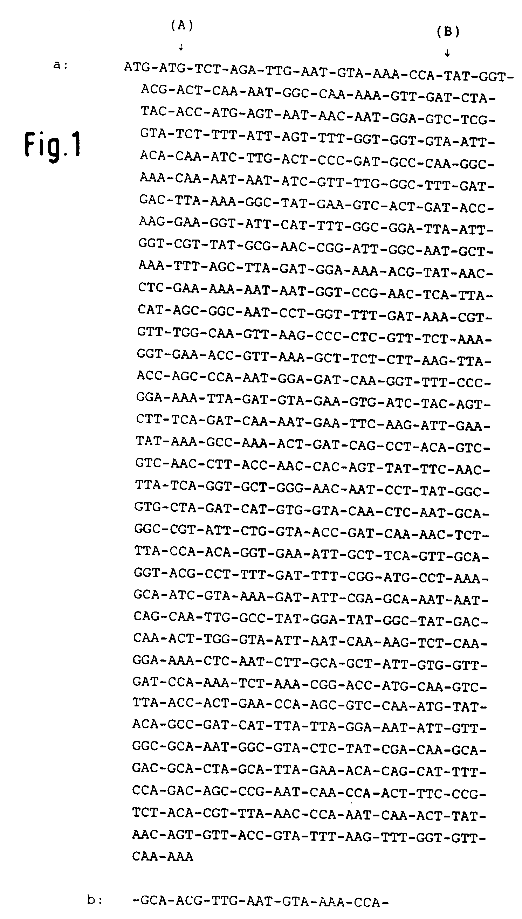

Gegenstand der Erfindung ist insbesondere eine für ein Polypeptid mit der biologischen Aktivität von Mutarotase codierende und dem Strukturgen entsprechende DNA-Sequenz, dadurch gekennzeichnet, daß sie die in Figur 1 (a, b) angegebene Nukleotidsequenz aufweist.

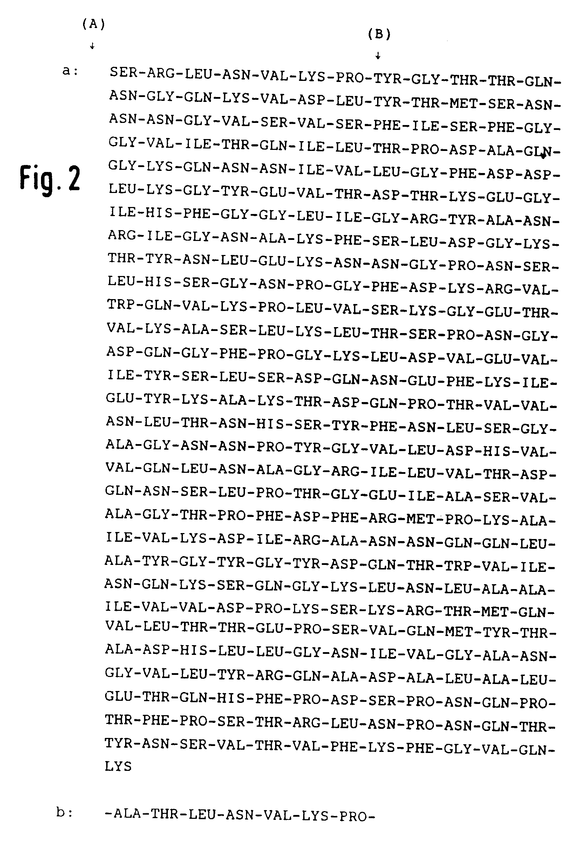

Gegenstand der Erfindung ist weiter ein Polypeptid mit der biologischen Aktivität von Mutarotase, dadurch gekennzeichnet, daß es die in Figur 2 (a, b) angegebene Aminosäure-Sequenz aufweist.

Gegenstand der Erfindung ist insbesondere auch das Expressionsplasmid mit der Bezeichnung pWH 1256 und der Hinterlegungsnummer DSM 4228 P.

Gegenstand der Erfindung ist ferner ein die erfindungsgemäße rekombinierte DNA enthaltender Wirtsorganismus, insbesondere E. coli WH 1256 mit der Hinterlegungsnummer DSM 4227.

Weiter ist Gegenstand der Erfindung ein Verfahren zur Herstellung eines Polypeptides mit der biologischen Aktivität von Mutarotase durch Kultivierung eines Mikroorganismus in einem Nährmedium und Isolierung des bei der Expression gebildeten Polypeptids, dadurch gekennzeichnet, daß man als Mikroorganismus einen mit mindestens einem erfindungsgemäßen rekombinierten DNA-Molekül transformierten Wirtsorganismus einsetzt.

Ferner ist Gegenstand der Erfindung die Verwendung eines Polypeptides mit der biologischen Aktivität von Mutarotase und den in Figur 2 (a, b) angegebenen Aminosäure-Sequenzen zur Beschleunigung enzymatischer Reaktionen von Aldosen.

Der Ausdruck "Polypeptid mit der biologischen Aktivität des Enzyms Mutarotase" bezeichnet ein Polypeptid bzw. Protein, dessen Aminosäure-Sequenz der nativen Mutarotase aus Acinetobacter calcoaceticus entspricht, ähnlich zu dieser Aminosäure-Sequenz ist oder nur einen Teilbereich davon umfaßt. Erfindungsgemäß werden auch entsprechende Fusionsproteine als "Polypeptide mit der biologischen Aktivität des Enzyms Mutarotase" bezeichnet.

Als Wirtsorganismen, in die Mutarotase codierende DNA eingebracht werden kann, kommen in erster Linie Mikroorganismen, aber auch pflanzliche, tierische oder menschliche Zellen in Frage. Bevorzugt sind Mikroorganismen wie Bakterien, Hefen und Fadenpilze. Besonders bevorzugt werden E. coli-Bakterien eingesetzt.

Tetracyclinresistenz-Gene einschließlich des Tetracyclin-Repressors sind ebenfalls bekannt (z.B. E. Winnacker, in: Gene und Klone, VCH (1985)). Erfindungsgemäß werden Tetracyclin-Repressor-Gene verwendet, die aus dem gleichen Organismus stammen wie der Promotor-Bereich unter dessen Kontrolle der gesamte codierende Bereich des Mutarotase-Gens steht.

Vorzugsweise werden also Promotor und Tetracyclin-Repressor-Gene aus E. coli eingesetzt.

Erfindungsgemäß eignen sich als Expressions-Kontroll-Sequenzen prinzipiell alle Promotoren, welche eine effektive Expression von Mutarotase bewirken. Vorzugsweise werden E. coli-Promotorsysteme wie z.B. das E. coli λ-P_{L}-Promotorsystem, das E. coli lac-System, das E. coli β-Lactamase-System, das E. coli trp-System oder das E. coli Lipoprotein-Promotorsystem verwendet. Besonders eignet sich der λ-P_{L}-Promotor, der z.B. aus der EP-OS-0041767 bekannt ist.

Zur Bereitstellung des erfindungsgemäßen gentechnologischen Verfahrens zur Herstellung eines Polypeptides mit der biologischen Aktivität von Mutarotase werden vorzugsweise folgende Schritte ausgeführt:

Ausgangsvektoren sind die Plasmide pWH 305 und pWH 1372. Das Plasmid pWH 305 und seine Konstruktion ist bei Oehmichen et al.,(1984) EMBO J. 3, 539-543 hinreichend beschrieben. pWH 305 enthält das Gen für den Tetracyclin-Repressor (tetR) und den λ-P_{L}-Promotor aus E. coli. pWH 1372 und seine Konstruktion ist aus der DE-OS-3531360 bekannt. pWH 1372 enthält eine DNA-Sequenz, codierend für ein Polypeptid mit der biologischen Aktivität von Mutarotase. Diese DNA-Sequenz stammt aus dem Genom von Acinetobacter calcoaceticus. Nukleotidsequenz der DNA und Aminosäuresequenz des Polypeptids sind in der DE-OS-3531360 dargelegt.

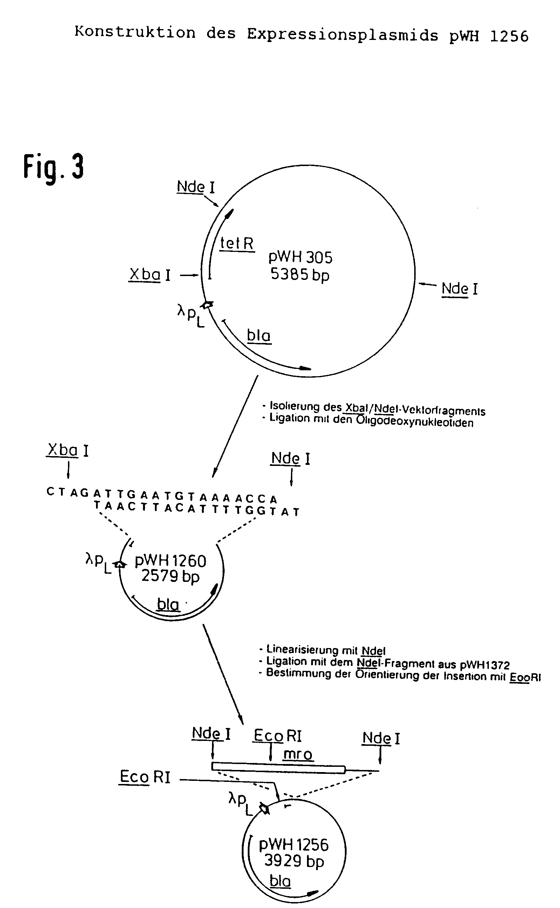

Zunächst wird pWH 305 am Genstart des tetR-Bereiches (Figur 3) und an etwa der gegenüberliegenden Seite mit bekannten Restriktionsenzymen nach Standardmethoden geschnitten. Für den Schnitt am Genstart von tetR eignet sich bevorzugt das bekannte Restriktionsenzym XbaI. Für die Schnittstelle auf der gegenüberliegenden Seite wird vorzugsweise die Restriktionsendonuklease NdeI eingesetzt. Durch die Schnittstellen entsteht unter anderem ein Vektorfragment, welches den λ-P_{L}-Promotor und das Ampicillin-Resistenzgen enthält. Dieses Vektorfragment wird anschließend mit einem Oligonukleotid-Doppelstrang der Sequenz I 
I      5'    CTAGATTGAATGTAAAACCA      3'
       3'       TAACTTACATTTTGGTAT    5'
in an sich bekannter Weise ligiert (Beispiel 1). Die Synthese der Oligonukleotide erfolgt nach Standardmethoden in an sich bekannter Weise bzw. wie in der DE-OS-3531360 ausführlich beschrieben. Es entsteht hieraus das neue Plasmid pWH 1260. Dieses Plasmid wird anschließend mit einer Restriktionsendonuklease, vorzugsweise NdeI, aufgeschnitten bzw. linearisiert. Die Restriktion von pWH 1372 erfolgt vorzugsweise mit NdeI etwa am Genstart sowie einige Nukleotide vom Genende des Mutarotase-Strukturgen entfernt. Die beiden Vektorfragmente werden in an sich bekannter Weise ligiert. Es entsteht so das neue erfindungsgemäße Expressionsplasmid pWH 1256 (Figur 3). Nach der Konstruktion des Expressionsvektors pWH 1256 wird dieser in üblicher Weise in einen transformierbaren Wirtsorganismus, vorzugsweise E. coli, eingebracht. Besonders geeignet ist beispielsweise der bekannte und gut zugängliche E. coli-Stamm Nr. 69 (E. coli K12ΔH1Δtrp), der auf seinem Chromosom einen Locus cI 857 enthält, der für den thermolabilen codiert. Daher wird in diesem Stamm die vom λ-P_{L}-Promotor gesteuerte Transkription bei 32 °C vollständig gehemmt, hingegen bei 40 °C nicht gehemmt, da bei dieser Temperatur der thermolabile Repressor cI 857 in seiner inaktiven Form vorliegt.

Anschließend wird der transformierte erfindungsgemäße Wirtsorganismus in an sich bekannte Weise in einem geeigneten Nährmedium kultiviert und das bei der Expression gebildete Polypeptid mit der biologischen Aktivität von Mutarotase wird daraus nach Standardmethoden gewonnen.

Erfindungsgemäß kann die Ligation des Vektorfragments aus pWH 305 und die weitere Konstruktion eines dem erfindungsgemäßen Vektor pWH 1256 verwandten, nicht näher bezeichneten Expressionsplasmids in oben beschriebener analoger Weise mit einem synthetisierten Doppelstrang-Oligonukleotid durchgeführt werden, welches partiell für eine Aminosäure-Sequenz codiert, die die in Figur 2b angegebene Teilsequenz aufweist. Die Expression von Mutarotase in entsprechend transformierten, nicht näher benannten E. coli-Wirtszellen führt zu Enzymaktivitäten, Ausbeuten und Eigenschaften, die sich mit den unten und in Beispiel 2 angegebenen Werten für das Polypeptid gemäß Figur 2a vergleichen lassen.

Alle dem oben geschilderten erfindungsgemäßen Verfahren zugrunde liegenden Methoden wie beispielsweise DNA-Restriktion, Ligation, Synthese von Oligonukleotiden, DNA- und Aminosäure-Sequenz-Analysen, Klonierung und Transformation, Isolierung und Aufreinigung des Expressionsproduktes sind durchweg in der Literatur gut beschrieben bzw. in der DE-OS-3531360 in detaillierter Form erläutert.

Die Nukleotidsequenz des erfindungsgemäßen hochexprimierten Strukturgens von Mutarotase gemäß Figur 1a (ausgehend von der synthetisierten Oligonukleotidsequenz I) entspricht der in der DE-OS-3531360 dargelegten Sequenz ab Nukleotid 80 (Position 28 Figur 1a). Im Vergleich zu dieser Sequenz enthält die erfindungsgemäße Sequenz von Figur 1a an Position 25 (Position 79 in der DE-OS-3531360) Cytosin (C) anstelle von Thymin (T). Zwischen Nukleotid-Position 60 und 70 (DE-OS-3531360) bzw. 6 und 16 (Figur 1a) hat sich die Sequenz von -GCA-ACG-TTA- zu -TCT-AGA-TTG verändert. Die ersten 60 Nukleotide (DE-OS-3531360) sind bei der erfindungsgemäßen Sequenz von Figur 1a ersetzt durch die Nukleotidfolge -ATGATG-.

Die Aminosäuresequenz ergibt sich demnach ab Nukleotid-Position 7 zu -Ser-Arg-Leu-Asn-Val-Lys-Pro- (Figur 2a) anstelle von -Ala-Thr-Leu-Asn-Val-Lys-Tyr (ab Nukleotid-Position 60 in der DE-OS-3531360).

Die ebenfalls erfindungsgemäße DNA-Sequenz von Figur 1b (ausgehend von der synthetisierten Oligonukleotidsequenz II) unterscheidet sich von der erfindungsgemäßen DNA-Sequenz gemäß Figur 1a lediglich zwischen Position 6 und 13 durch die Sequenz -GCA-ACG- anstelle von -TCT-AGA-. Entsprechend ändert sich die Aminosäuresequenz -Ser-Arg- zu -Ala-Thr- (Figur 2b). Die Veränderungen sind teilweise auch in Figur 4 erläutert.

Die geschilderten Änderungen bewirken, daß durch das erfindungsgemäße Verfahren ein Polypeptid mit der biologischen Aktivität von Mutarotase mit überraschend hohen Ausbeuten bereitgestellt wird. So können z.B. in einem entsprechend transformierten E. coli-Wirt, Mutarotase-Aktivitäten von 40.000 bis 200.000, vorzugsweise von 100.000 bis 150.000 Units/l Kulturbrühe erzielt werden.

Dies ist eine etwa 30fache Steigerung an Enzymaktivität gegenüber dem in der DE-OS-3531360 beschriebenen Verfahren. Ein Vergleich mit Mutarotase-Aktivitäten in zum Teil heute noch verwendeten optimierten Stämmen von Aspergillus niger bzw. Acinetobacter calcoaceticus, erhältlich durch klassische mikrobielle Verfahren, ergibt sogar eine Steigerung um etwa den Faktor 30.000 bzw. 3.000. Die Mengenausbeuten des erfindungsgemäßen Verfahrens variieren zwischen 200 und 800, vorzugsweise zwischen 500 und 650 mg Mutarotase/l Kulturmedium.

Die nach dem erfindungsgemäßen Verfahren in E. coli exprimierte Mutarotase läßt sich ebenso einfach und effektiv isolieren und aufreinigen, wie es in der DE-OS-3531360 beschrieben wurde. Sie weist ebenfalls die vorteilhaften nahezu identischen Eigenschaften auf.

Aufgrund dieser guten Eigenschaften und ihrer sehr hohen Aktivität ist die nach dem erfindungsgemäßen Verfahren hergestellte Mutarotase vorzüglich geeignet zur Beschleunigung enzymatischer Reaktionen von Aldosen.

Im folgenden sind die Abbildungen erläutert, in denen Teilbereiche der Erfindung übersichtlich dargestellt sind.
- Figur 1:: DNA-Sequenz des Strukturgen-Bereiches, codierend für ein Polypeptid mit der biologischen Aktivität von Mutarotase
a: DNA-Sequenz, vorliegend im Plasmid pWH 1256
b: zwischen den Positionen (A) und (B) alternative Sequenz
- Figur 2:: a: Aminosäuresequenz des Expressionsproduktes von pWH 1256
b: zwischen den Positionen (A) und (B) alternative Sequenz
- Figur 3:: Konstruktion des Expressionsplasmids pWH 1256 aus den Plasmiden pWH 305 und pWH 1372.
pWH 305 enthält den tetR-Bereich und den A-P_{L}-Promotor aus E. coli. pWH 1372 enthält einen Mutarotase codierenden Genbereich aus Acinetobacter calcoaceticus.

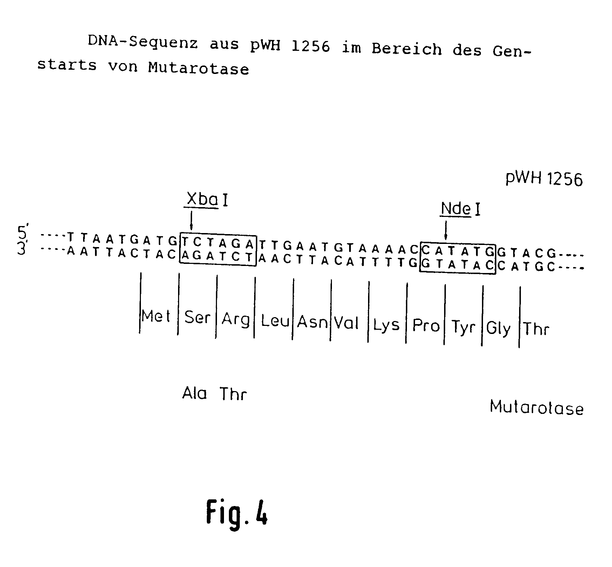

- Figur 4:: Nukleotid-Sequenz aus pWH 1256 im Bereich des Genstarts von Mutarotase. Eingezeichnet sind die Schnittstellen der vorzugsweise verwendeten Restriktionsenzyme XbaI und NdeI sowie die zugeordneten Aminosäuren.

### Beispiel 1

### Konstruktion des Expressionsplasmids pWH 1256 und Transformation von E. coli

19 µg pWH 305 (Oehmichen et al., l.c.) werden mit den Restriktionsendonukleasen XbaI und NdeI restringiert und das Vektorfragment nach Elektrophorese aus einem Agarosegel eluiert.

Zwei Oligonukleotide mit den Sequenzen 
5' CTAGATTGAATGTAAAACCA 3'
3' TAACTTACATTTTGGTAT 5'
werden wie in der DE-OS-3531360 beschrieben synthetisiert und in einem 100fachen molaren Überschuß mit dem Vektorfragment ligiert. Das Reaktionsprodukt wird auf E. coli W6 in an sich bekannter Weise übertragen. E. coli W6 ist aus der EP-OS-0041767 bekannt. Aus 50 Ampicillin resistenten Kandidaten wird die Plasmid DNA im Schnellaufschluß präpariert und durch Restriktion mit XbaI und NdeI untersucht. Ein Kolonie, deren Plasmid von beiden Restriktionsendonukleasen linearisiert wird, wird durch Doppelstrang-Didesoxysequenzierung charakterisiert (Chen u. Seeburg (1985), DNA 4; 165-170). Dabei wird die obige Sequenz des Oligonukleotids sowie die Sequenzen der flankierenden Vektorbereiche (Postle et al. (1984), Nucleic Acids Res. 12, 4849-4863; Sutcliffe (1979). Cold Spring Harbor Symp. Quant. Biol. 43, 77-90) bestätigt. Das entstandene Plasmid trägt die Bezeichnung pWH 1260 und wird aus 1 l Kultur präpariert. 5 µg pWH 1260 werden mit der Restriktionsendonuklease NdeI linearisiert. 10 µg pWH 1372 (DE-OS-3531360) werden mit der Restriktionsendonuklease NdeI verdaut und das 1350 bp lange Fragment aus einem 1 % Agarosegel in üblicher Weise eluiert. Equimolare Mengen linearisierter pWH 1260-DNA und das 1350 bp Fragment aus pWH 1372 werden ligiert und auf E. coli W6 übertragen. Plasmid-DNA aus fünfzig Ampicillin resistenten Kandidaten wird im Schnellaufschluß präpariert und auf erfolgte Insertion durch Elektrophorese auf 1 % Agarose überprüft. Rekombinante Kolonien werden durch Restriktion mit EcoRI auf die Insertionsrichtung überprüft. Das Plasmid mit der tetR (pWH 305) mro (pWH 1372) Fusion wird pWH 1256 genannt und aus 1 l Kultur präpariert. Zur Expression des Fusionsproteins wird das Plasmid auf E. coli K12ΔH1Δtrp (DE-OS-3531360) bei 30 °C transformiert. Der entstehende Stamm trägt die Bezeichnung E. coli WH 1256. Die Konstruktion von pWH 1256 ist in Figur 3 schematisch dargestellt.

### Beispiel 2

### Expression von Mutarotase mit dem Expressionsplasmid pWH 1256 in E. coli 69

Der E. coli-Stamm Nr. 69 (E. coli K12ΔH1Δtrp) wird mit dem rekombinanten Expressionsplasmid pWH 1256 nach Standardmethoden transformiert. Dieser Wirtsbakterienstamm enthält auf seinem Chromosom einen Locus c 857, der für den thermolabilen Repressorcodiert. Daher wird in diesem Stamm die vom λ-P_{L}-Promotor gesteuerte Transkription bei 32 °C vollständig gehemmt, hingegen bei 40 °C nicht gehemmt, da bei dieser Temperatur der thermolabile Repressor I 857 in seiner inaktiven Form vorliegt.

In einem typischen Expressionsverfahren werden in einem Fermenter 4 l LB-Medium (10 g/l Trypton, 8 g/l NaCl, 5 g/l Hefeextrakt, pH 7,8, 0,1 g/l Ampicillin) mit 200 ml einer Übernachtkultur von E. coli 69/pWH 1256 angeimpft und bei 28 °C bis zu einer Zelldichte von 5 OD (650 nm) kultiviert. Anschließend wird die Kultur auf 42 °C erhitzt und weiter belüftet. Die Mutarotase-Aktivität wird nach dem folgenden Zellaufschluß nachgewiesen.

1 ml Zellkultur wird entnommen, auf 50 mM EDTA eingestellt, 10 min auf Eis inkubiert mit 100 µl Lysozym (10 mg/ml) versetzt und weitere 10 min auf Eis inkubiert. Anschließend werden die Zellen unter Eiskühlung lysiert. Dann werden die Zelltrümmer abzentrifugiert und der Überstand wie oben angegeben auf Mutarotase-Aktivität untersucht. Die gemessenen Expressionswerte von 150.000 U/l Kulturmedium entsprechen etwa 650 mg Mutarotase/l Kulturmedium.

## Patentansprüche

1. Rekombinante DNA-Sequenz, codierend für ein Polypeptid mit der biologischen Aktivität von Mutarotase, dadurch gekennzeichnet, daß sie das Strukturgen aus dem Genom eines Mutarotase bildenden Mikroorganismus, den Genstart des Tetracyclin-Repressors und eine Promotor-Sequenz enthält, wobei der sequenzbereich des Tetracyclin-Repressors und die Promotor-Sequenz aus dem Mikroorganismus stammen, in dem die Expression des Strukturgens durchgeführt wird.

2. DNA-Sequenz nach Anspruch 1, dadurch gekennzeichnet, daß das Strukturgen die Nukleotidsequenz 
(A)                                    (B)
ATG-ATG-TCT-AGA-TTG-AAT-GTA-AAA-CCA-TAT-GGT-ACG-
ACT-CAA-AAT-GGC-CAA-AAA-GTT-GAT-CTA-TAC-ACC-ATG-
AGT-AAT-AAC-AAT-GGA-GTC-TCG-GTA-TCT-TTT-ATT-AGT-
TTT-GGT-GGT-GTA-ATT-ACA-CAA-ATC-TTG-ACT-CCC-GAT-
GCC-CAA-GGC-AAA-CAA-AAT-AAT-ATC-GTT-TTG-GGC-TTT-
GAT-GAC-TTA-AAA-GGC-TAT-GAA-GTC-ACT-GAT-ACC-AAG-
GAA-GGT-ATT-CAT-TTT-GGC-GGA-TTA-ATT-GGT-CGT-TAT-
GCG-AAC-CGG-ATT-GGC-AAT-GCT-AAA-TTT-AGC-TTA-GAT-
GGA-AAA-ACG-TAT-AAC-CTC-GAA-AAA-AAT-AAT-GGT-CCG-
AAC-TCA-TTA-CAT-AGC-GGC-AAT-CCT-GGT-TTT-GAT-AAA-
CGT-GTT-TGG-CAA-GTT-AAG-CCC-CTC-GTT-TCT-AAA-GGT-
 
GAA-ACC-GTT-AAA-GCT-TCT-CTT-AAG-TTA-ACC-AGC-CCA-
AAT-GGA-GAT-CAA-GGT-TTT-CCC-GGA-AAA-TTA-GAT-GTA-
GAA-GTG-ATC-TAC-AGT-CTT-TCA-GAT-CAA-AAT-GAA-TTC-
AAG-ATT-GAA-TAT-AAA-GCC-AAA-ACT-GAT-CAG-CCT-ACA-
GTC-GTC-AAC-CTT-ACC-AAC-CAC-AGT-TAT-TTC-AAC-TTA-
TCA-GGT-GCT-GGG-AAC-AAT-CCT-TAT-GGC-GTG-CTA-GAT-
CAT-GTG-GTA-CAA-CTC-AAT-GCA-GGC-CGT-ATT-CTG-GTA-
ACC-GAT-CAA-AAC-TCT-TTA-CCA-ACA-GGT-GAA-ATT-GCT-
TCA-GTT-GCA-GGT-ACG-CCT-TTT-GAT-TTT-CGG-ATG-CCT-
AAA-GCA-ATC-GTA-AAA-GAT-ATT-CGA-GCA-AAT-AAT-CAG-
CAA-TTG-GCC-TAT-GGA-TAT-GGC-TAT-GAC-CAA-ACT-TGG-
GTA-ATT-AAT-CAA-AAG-TCT-CAA-GGA-AAA-CTC-AAT-CTT-
GCA-GCT-ATT-GTG-GTT-GAT-CCA-AAA-TCT-AAA-CGG-ACC-
ATG-CAA-GTC-TTA-ACC-ACT-GAA-CCA-AGC-GTC-CAA-ATG-
TAT-ACA-GCC-GAT-CAT-TTA-TTA-GGA-AAT-ATT-GTT-GGC-
GCA-AAT-GGC-GTA-CTC-TAT-CGA-CAA-GCA-GAC-GCA-CTA-
GCA-TTA-GAA-ACA-CAG-CAT-TTT-CCA-GAC-AGC-CCG-AAT-
CAA- CCA -ACT-TTC-CCG-TCT-ACA-CGT-TTA-AAC-CCA-AAT-
CAA-ACT-TAT-AAC-AGT-GTT-ACC-GTA-TTT-AAG-TTT-GGT-
GTT-CAA-AAA
 aufweist.

3. DNA-Sequenz nach Anspruch 1 und 2, dadurch gekennzeichnet, daß sie zwischen den Positionen (A) und (B) die Nukleotidsequenz -GCA-ACG-TTG-AAT-GTA-AAA-CCA- aufweist.

4. Polypeptid mit der biologischen Aktivität von Mutarotase, dadurch gekennzeichnet, daß es die Aminosäure-Sequenz 
(A)                                    (B)
↓                                      ↓
-SER-ARG-LEU-ASN-VAL-LYS-PRO-TYR-GLY-THR-THR-GLN-
ASN-GLY-GLN-LYS-VAL-ASP-LEU-TYR-THR-MET-SER-ASN-
ASN-ASN-GLY-VAL-SER-VAL-SER-PHE-ILE-SER-PHE-GLY-
GLY-VAL-ILE-THR-GLN-ILE-LEU-THR-PRO-ASP-ALA-GLN-
GLY-LYS-GLN-ASN-ASN-ILE-VAL-LEU-GLY-PHE-ASP-ASP-
LEU-LYS-GLY-TYR-GLU-VAL-THR-ASP-THR-LYS-GLU-GLY-
ILE-HIS-PHE-GLY-GLY-LEU-ILE-GLY-ARG-TYR-ALA-ASN-
ARG-ILE-GLY-ASN-ALA-LYS-PHE-SER-LEU-ASP-GLY-LYS-
THR-TYR-ASN-LEU-GLU-LYS-ASN-ASN-GLY-PRO-ASN-SER-
LEU-HIS-SER-GLY-ASN-PRO-GLY-PHE-ASP-LYS-ARG-VAL-
TRP-GLN-VAL-LYS-PRO-LEU-VAL-SER-LYS-GLY-GLU-THR-
VAL-LYS-ALA-SER-LEU-LYS-LEU-THR-SER-PRO-ASN-GLY-
ASP-GLN-GLY-PHE-PRO-GLY-LYS-LEU-ASP-VAL-GLU-VAL-
ILE-TYR-SER-LEU-SER-ASP-GLN-ASN-GLU-PHE-LYS-ILE-
GLU-TYR-LYS-ALA-LYS-THR-ASP-GLN-PRO-THR-VAL-VAL-
ASN-LEU-THR-ASN-HIS-SER-TYR-PHE-ASN-LEU-SER-GLY-
ALA-GLY-ASN-ASN-PRO-TYR-GLY-VAL-LEU-ASP-HIS-VAL-
VAL-GLN-LEU-ASN-ALA-GLY-ARG-ILE-LEU-VAL-THR-ASP-
GLN-ASN-SER-LEU-PRO-THR-GLY-GLU-ILE-ALA-SER-VAL-
ALA-GLY-THR-PRO-PHE-ASP-PHE-ARG-MET-PRO-LYS-ALA-
ILE-VAL-LYS-ASP-ILE-ARG-ALA-ASN-ASN-GLN-GLN-LEU-
ALA-TYR-GLY-TYR-GLY-TYR-ASP-GLN-THR-TRP-VAL-ILE-
ASN-GLN-LYS-SER-GLN-GLY-LYS-LEU-ASN-LEU-ALA-ALA-
ILE-VAL-VAL-ASP-PRO-LYS-SER-LYS-ARG-THR-MET-GLN-
VAL-LEU-THR-THR-GLU-PRO-SER-VAL-GLN-MET-TYR-THR-
ALA-ASP-HIS-LEU-LEU-GLY-ASN-ILE-VAL-GLY-ALA-ASN-
GLY-VAL-LEU-TYR-ARG-GLN-ALA-ASP-ALA-LEU-ALA-LEU-
 
GLU-THR-GLN-HIS-PHE-PRO-ASP-SER-PRO-ASN-GLN-PRO-
THR-PHE-PRO-SER-THR-ARG-LEU-ASN-PRO-ASN-GLN-THR-
TYR-ASN-SER-VAL-THR-VAL-PHE-LYS-PHE-GLY-VAL-GLN-
LYS
 aufweist.

5. Polypeptid mit der biologischen Aktivität von Mutarotase, dadurch gekennzeichnet, daß es zwischen den Positionen (A) und (B) die Polypeptid-Sequenz -ALA-THR-LEU-ASN-VAL-LYS-PRO- aufweist.

6. Polypeptid mit der biologischen Aktivität des Enzyms Mutarotase, dadurch gekennzeichnet, daß es von der DNA-Sequenz gemäß Anspruch 2 oder 3 codiert wird.

7. DNA-Sequenz nach einem der Ansprüche 1 bis 3, dadurch gekennzeichnet, daß das Strukturgen aus dem Genom der Gattung Acinetobacter stammt.

8. DNA-Sequenz nach einem der Ansprüche 1 bis 3, dadurch gekennzeichnet, daß die Sequenz des Tetracyclin-Repressors aus E. coli stammt.

9. DNA-Sequenz nach einem der Ansprüche 1 bis 3, dadurch gekennzeichnet, daß die Promotor-Sequenz aus E. coli stammt.

10. DNA-Sequenz nach Anspruch 1, dadurch gekennzeichnet, daß das Strukturgen die Nucleotid-Sequenz zwischen den Positionen (A) und (B) der DNA-Sequenz gemäß Anspruch 2 oder 3 aufweist oder eine Nucleotid-Sequenz, die mit dieser durch gezielte oder ungezielte Variationen verwandt ist oder mit ihr hybridisiert.

11. Expressionsplasmid mit der Bezeichnung pWH 1256 und der Hinterlegungsnummer DSM 4228 P.

12. Wirtsorganismus, dadurch gekennzeichnet, da er mit mindestens einem DNA-Molekül nach einem der Ansprüche 1 bis 3 transformiert ist.

13. Wirtsorganismus nach Anspruch 12, dadurch gekennzeichnet, daß er E. coli ist.

14. Wirtsorganismus mit der Bezeichnung E. coli WH 1256 und der Hinterlegungsnummer DSM 4227.

15. Verfahren zur Herstellung eines Polypeptids mit der biologischen Aktivität von Mutarotase durch Kultivierung eines Mikroorganismus in einem Nährmedium und Isolierung des bei der Expression gebildeten Polypeptids, dadurch gekennzeichnet, daß man als Mikroorganismus einen mit mindestens einem rekombinanten DNA-Molekül gemäß Anspruch 1 oder 10 transformierten Wirtsorganismus einsetzt.

16. Verwendung eines Polypeptids nach Anspruch 4, 5 oder 6 zur Beschleunigung enzymatischer Reaktionen von Aldosen.

## Claims

1. Recombinant DNA sequence coding for a polypeptide having the biological activity of mutarotase, characterised in that it contains the structural gene from the genome of a mutarotase-producing microorganism, the gene start of the tetracycline-repressor and a promoter sequence, with the tetracycline-repressor sequence region and the promoter sequence originating from the microorganism in which the expression of the structural gene is carried out.

2. DNA sequence according to Claim 1, characterised in that the structural gene has the nucleotide sequence

(A)                                    (B)
↓                                      ↓
ATG-ATG-TCT-AGA-TTG-AAT-GTA-AAA-CCA-TAT-GGT-ACG-
ACT-CAA-AAT-GGC-CAA-AAA-GTT-GAT-CTA-TAC-ACC-ATG-
AGT-AAT-AAC-AAT-GGA-GTC-TCG-GTA-TCT-TTT-ATT-AGT-
TTT-GGT-GGT-GTA-ATT-ACA-CAA-ATC-TTG-ACT-CCC-GAT-
GCC-CAA-GGC-AAA-CAA-AAT-AAT-ATC-GTT-TTG-GGC-TTT-
GAT-GAC-TTA-AAA-GGC-TAT-GAA-GTC-ACT-GAT-ACC-AAG-
GAA-GGT-ATT-CAT-TTT-GGC-GGA-TTA-ATT-GGT-CGT-TAT-
GCG-AAC-CGG-ATT-GGC-AAT-GCT-AAA-TTT-AGC-TTA-GAT-
GGA-AAA-ACG-TAT-AAC-CTC-GAA-AAA-AAT-AAT-GGT-CCG-
AAC-TCA-TTA-CAT-AGC-GGC-AAT-CCT-GGT-TTT-GAT-AAA-
CGT-GTT-TGG-CAA-GTT-AAG-CCC-CTC-GTT-TCT-AAA-GGT-
GAA-ACC-GTT-AAA-GCT-TCT-CTT-AAG-TTA-ACC-AGC-CCA-
AAT-GGA-GAT-CAA-GGT-TTT-CCC-GGA-AAA-TTA-GAT-GTA-
GAA-GTG-ATC-TAC-AGT-CTT-TCA-GAT-CAA-AAT-GAA-TTC-
AAG-ATT-GAA-TAT-AAA-GCC-AAA-ACT-GAT-CAG-CCT-ACA-
GTC-GTC-AAC-CTT-ACC-AAC-CAC-AGT-TAT-TTC-AAC-TTA-
TCA-GGT-GCT-GGG-AAC-AAT-CCT-TAT-GGC-GTG-CTA-GAT-
CAT-GTG-GTA-CAA-CTC-AAT-GCA-GGC-CGT-ATT-CTG-GTA-
ACC-GAT-CAA-AAC-TCT-TTA-CCA-ACA-GGT-GAA-ATT-GCT-
TCA-GTT-GCA-GGT-ACG-CCT-TTT-GAT-TTT-CGG-ATG-CCT-

AAA-GCA-ATC-GTA-AAA-GAT-ATT-CGA-GCA-AAT-AAT-CAG-
CAA-TTG-GCC-TAT-GGA-TAT-GGC-TAT-GAC-CAA-ACT-TGG-
GTA-ATT-AAT-CAA-AAG-TCT-CAA-GGA-AAA-CTC-AAT-CTT-
GCA-GCT-ATT-GTG-GTT-GAT-CCA-AAA-TCT-AAA-CGG-ACC-
ATG-CAA-GTC-TTA-ACC-ACT-GAA-CCA-AGC-GTC-CAA-ATG-
TAT-ACA-GCC-GAT-CAT-TTA-TTA-GGA-AAT-ATT-GTT-GGC-
GCA-AAT-GGC-GTA-CTC-TAT-CGA-CAA-GCA-GAC-GCA-CTA-
GCA-TTA-GAA-ACA-CAG-CAT-TTT-CCA-GAC-AGC-CCG-AAT-
CCA
CAA-    -ACT-TTC-CCG-TCT-ACA-CGT-TTA-AAC-CCA-AAT-
CAA-ACT-TAT-AAC-AGT-GTT-ACC-GTA-TTT-AAG-TTT-GGT-
GTT-CAA-AAA

3. DNA sequence according to Claim 1 and 2, characterised in that it has between positions (A) and (B) the nucleotide sequence

-GCA-ACG-TTG-AAT-GTA-AAA-CCA-

4. Polypeptide having the biological activity of mutarotase, characterised in that it has the amino acid sequence

(A)
↓
(B)
↓
-SER-ARG-LEU-ASN-VAL-LYS-PRO-TYR-GLY-THR-THR-GLN-
ASN-GLY-GLN-LYS-VAL-ASP-LEU-TYR-THR-MET-SER-ASN-
ASN-ASN-GLY-VAL-SER-VAL-SER-PHE-ILE-SER-PHE-GLY-
GLY-VAL-ILE-THR-GLN-ILE-LEU-THR-PRO-ASP-ALA-GLN-
GLY-LYS-GLN-ASN-ASN-ILE-VAL-LEU-GLY-PHE-ASP-ASP-
LEU-LYS-GLY-TYR-GLU-VAL-THR-ASP-THR-LYS-GLU-GLY-
ILE-HIS-PHE-GLY-GLY-LEU-ILE-GLY-ARG-TYR-ALA-ASN-
ARG-ILE-GLY-ASN-ALA-LYS-PHE-SER-LEU-ASP-GLY-LYS-
THR-TYR-ASN-LEU-GLU-LYS-ASN-ASN-GLY-PRO-ASN-SER-
LEU-HIS-SER-GLY-ASN-PRO-GLY-PHE-ASP-LYS-ARG-VAL-
TRP-GLN-VAL-LYS-PRO-LEU-VAL-SER-LYS-GLY-GLU-THR-
VAL-LYS-ALA-SER-LEU-LYS-LEU-THR-SER-PRO-ASN-GLY-
ASP-GLN-GLY-PHE-PRO-GLY-LYS-LEU-ASP-VAL-GLU-VAL-
ILE-TYR-SER-LEU-SER-ASP-GLN-ASN-GLU-PHE-LYS-ILE-
GLU-TYR-LYS-ALA-LYS-THR-ASP-GLN-PRO-THR-VAL-VAL-
ASN-LEU-THR-ASN-HIS-SER-TYR-PHE-ASN-LEU-SER-GLY-
ALA-GLY-ASN-ASN-PRO-TYR-GLY-VAL-LEU-ASP-HIS-VAL-
VAL-GLN-LEU-ASN-ALA-GLY-ARG-ILE-LEU-VAL-THR-ASP-
GLN-ASN-SER-LEU-PRO-THR-GLY-GLU-ILE-ALA-SER-VAL-
ALA-GLY-THR-PRO-PHE-ASP-PHE-ARG-MET-PRO-LYS-ALA-
ILE-VAL-LYS-ASP-ILE-ARG-ALA-ASN-ASN-GLN-GLN-LEU-
ALA-TYR-GLY-TYR-GLY-TYR-ASP-GLN-THR-TRP-VAL-ILE-
ASN-GLN-LYS-SER-GLN-GLY-LYS-LEU-ASN-LEU-ALA-ALA-
ILE-VAL-VAL-ASP-PRO-LYS-SER-LYS-ARG-THR-MET-GLN-
VAL-LEU-THR-THR-GLU-PRO-SER-VAL-GLN-MET-TYR-THR-
ALA-ASP-HIS-LEU-LEU-GLY-ASN-ILE-VAL-GLY-ALA-ASN-
GLY-VAL-LEU-TYR-ARG-GLN-ALA-ASP-ALA-LEU-ALA-LEU-
GLU-THR-GLN-HIS-PHE-PRO-ASP-SER-PRO-ASN-GLN-PRO-
THR-PHE-PRO-SER-THR-ARG-LEU-ASN-PRO-ASN-GLN-THR-
TYR-ASN-SER-VAL-THR-VAL-PHE-LYS-PHE-GLY-VAL-GLN-
LYS

5. Polypeptide having the biological activity of mutarotase, characterised in that it has between positions (A) and (B) the polypeptide sequence

-ALA-THR-LEU-ASN-VAL-LYS-PRO-

6. Polypeptide having the biological activity of the enzyme mutarotase, characterised in that it is encoded by the DNA sequence according to Claim 2 or 3.

7. DNA sequence according to any of Claims 1 to 3, characterised in that the structural gene originates from the genome of the genus Acinetobacter.

8. DNA sequence according to any of Claims 1 to 3, characterised in that the sequence of the tetracycline repressor originates from E. coli.

9. DNA sequence according to any of Claims 1 to 3, characterised in that the promoter sequence originates from E. coli.

10. DNA sequence according to Claim 1, characterised in that the structural gene has the nucleotide sequence between positions (A) and (B) of the DNA sequence according to Claim 2 or 3, or a nucleotide sequence which is related to this by specific or non-specific modifications, or hybridises with it.

11. Expression plasmid with the designation pWH 1256 and the deposit number DSM 4228 P.

12. Host organism characterised in that it is transformed with at least one DNA molecule according to any of Claims 1 to 3.

13. Host organism according to Claim 12, characterised in that it is E. coli.

14. Host organism with the designation E. coli WH 1256 and the deposit number DSM 4227.

15. Process for preparing a polypeptide having the biological activity of mutarotase by cultivation of a microorganism in a nutrient medium and isolation of the polypeptide formed by expression, characterised in that the microorganism used is a host organism transformed with at least one recombinant DNA molecule according to Claim 1 or 10.

16. Use of a polypeptide according to Claim 4, 5 or 6 for increasing the rate of enzymatic reactions of aldoses.

## Revendications

1. Séquence d'ADN recombinant, codant pour un polypeptide ayant l'activité biologique de la mutarotase, caractérisée en ce qu'elle contient le gène structural provenant du génome d'un micro-organisme producteur de mutarotase, l'initiateur du répresseur de la tétracycline et une séquence promotrice, la région de séquence du répresseur de la tétracycline et la séquence promotrice provenant du micro-organisme dans lequel est effectuée l'expression du gène structural.

2. Séquence d'ADN selon la revendication 1, caractérisée en ce que le gène structural comporte la séquence nucléotidique

(A)                                    (B)
↓                                      ↓
ATG-ATG-TCT-AGA-TTG-AAT-GTA-AAA-CCA-TAT-GGT-ACG-
ACT-CAA-AAT-GGC-CAA-AAA-GTT-GAT-CTA-TAC-ACC-ATG-
AGT-AAT-AAC-AAT-GGA-GTC-TCG-GTA-TCT-TTT-ATT-AGT-
TTT-GGT-GGT-GTA-ATT-ACA-CAA-ATC-TTG-ACT-CCC-GAT-
GCC-CAA-GGC-AAA-CAA-AAT-AAT-ATC-GTT-TTG-GGC-TTT-
GAT-GAC-TTA-AAA-GGC-TAT-GAA-GTC-ACT-GAT-ACC-AAG-
GAA-GGT-ATT-CAT-TTT-GGC-GGA-TTA-ATT-GGT-CGT-TAT-
GCG-AAC-CGG-ATT-GGC-AAT-GCT-AAA-TTT-AGC-TTA-GAT-
GGA-AAA-ACG-TAT-AAC-CTC-GAA-AAA-AAT-AAT-GGT-CCG-
AAC-TCA-TTA-CAT-AGC-GGC-AAT-CCT-GGT-TTT-GAT-AAA-
CGT-GTT-TGG-CAA-GTT-AAG-CCC-CTC-GTT-TCT-AAA-GGT-
GAA-ACC-GTT-AAA-GCT-TCT-CTT-AAG-TTA-ACC-AGC-CCA-
AAT-GGA-GAT-CAA-GGT-TTT-CCC-GGA-AAA-TTA-GAT-GTA-
GAA-GTG-ATC-TAC-AGT-CTT-TCA-GAT-CAA-AAT-GAA-TTC-
AAG-ATT-GAA-TAT-AAA-GCC-AAA-ACT-GAT-CAG-CCT-ACA-
GTC-GTC-AAC-CTT-ACC-AAC-CAC-AGT-TAT-TTC-AAC-TTA-
TCA-GGT-GCT-GGG-AAC-AAT-CCT-TAT-GGC-GTG-CTA-GAT-
CAT-GTG-GTA-CAA-CTC-AAT-GCA-GGC-CGT-ATT-CTG-GTA-
ACC-GAT-CAA-AAC-TCT-TTA-CCA-ACA-GGT-GAA-ATT-GCT-
TCA-GTT-GCA-GGT-ACG-CCT-TTT-GAT-TTT-CGG-ATG-CCT-

AAA-GCA-ATC-GTA-AAA-GAT-ATT-CGA-GCA-AAT-AAT-CAG-
CAA-TTG-GCC-TAT-GGA-TAT-GGC-TAT-GAC-CAA-ACT-TGG-
GTA-ATT-AAT-CAA-AAG-TCT-CAA-GGA-AAA-CTC-AAT-CTT-
GCA-GCT-ATT-GTG-GTT-GAT-CCA-AAA-TCT-AAA-CGG-ACC-
ATG-CAA-GTC-TTA-ACC-ACT-GAA-CCA-AGC-GTC-CAA-ATG-
TAT-ACA-GCC-GAT-CAT-TTA-TTA-GGA-AAT-ATT-GTT-GGC-
GCA-AAT-GGC-GTA-CTC-TAT-CGA-CAA-GCA-GAC-GCA-CTA-
GCA-TTA-GAA-ACA-CAG-CAT-TTT-CCA-GAC-AGC-CCG-AAT-
CCA
CAA-[?]-ACT-TTC-CCG-TCT-ACA-CGT-TTA-AAC-CCA-AAT-
CAA-ACT-TAT-AAC-AGT-GTT-ACC-GTA-TTT-AAG-TTT-GGT-
GTT-CAA-AAA

3. Séquence d'ADN selon les revendications 1 et 2, caractérisée en ce qu'elle comporte, entre les positions (A) et (B), la séquence nucléotidique

-GCA-ACG-TTG-AAT-GTA-AAA-CCA-

4. Polypeptide ayant l'activité biologique de la mutarotase, caractérisé en ce qu'il comporte la séquence d'aminoacides

(A)                    (B)
↓                      ↓
-SER-ARG-LEU-ASN-VAL-LYS-PRO-TYR-GLY-THR-THR-GLN-
ASN-GLY-GLN-LYS-VAL-ASP-LEU-TYR-THR-MET-SER-ASN-
ASN-ASN-GLY-VAL-SER-VAL-SER-PHE-ILE-SER-PHE-GLY-
GLY-VAL-ILE-THR-GLN-ILE-LEU-THR-PRO-ASP-ALA-GLN-
GLY-LYS-GLN-ASN-ASN-ILE-VAL-LEU-GLY-PHE-ASP-ASP-
LEU-LYS-GLY-TYR-GLU-VAL-THR-ASP-THR-LYS-GLU-GLY-
ILE-HIS-PHE-GLY-GLY-LEU-ILE-GLY-ARG-TYR-ALA-ASN-
ARG-ILE-GLY-ASN-ALA-LYS-PHE-SER-LEU-ASP-GLY-LYS-
THR-TYR-ASN-LEU-GLU-LYS-ASN-ASN-GLY-PRO-ASN-SER-
LEU-HIS-SER-GLY-ASN-PRO-GLY-PHE-ASP-LYS-ARG-VAL-
TRP-GLN-VAL-LYS-PRO-LEU-VAL-SER-LYS-GLY-GLU-THR-
VAL-LYS-ALA-SER-LEU-LYS-LEU-THR-SER-PRO-ASN-GLY-
ASP-GLN-GLY-PHE-PRO-GLY-LYS-LEU-ASP-VAL-GLU-VAL-
ILE-TYR-SER-LEU-SER-ASP-GLN-ASN-GLU-PHE-LYS-ILE-
GLU-TYR-LYS-ALA-LYS-THR-ASP-GLN-PRO-THR-VAL-VAL-

ASN-LEU-THR-ASN-HIS-SER-TYR-PHE-ASN-LEU-SER-GLY-
ALA-GLY-ASN-ASN-PRO-TYR-GLY-VAL-LEU-ASP-HIS-VAL-
VAL-GLN-LEU-ASN-ALA-GLY-ARG-ILE-LEU-VAL-THR-ASP-
GLN-ASN-SER-LEU-PRO-THR-GLY-GLU-ILE-ALA-SER-VAL-
ALA-GLY-THR-PRO-PHE-ASP-PHE-ARG-MET-PRO-LYS-ALA-
ILE-VAL-LYS-ASP-ILE-ARG-ALA-ASN-ASN-GLN-GLN-LEU-
ALA-TYR-GLY-TYR-GLY-TYR-ASP-GLN-THR-TRP-VAL-ILE-
ASN-GLN-LYS-SER-GLN-GLY-LYS-LEU-ASN-LEU-ALA-ALA-
ILE-VAL-VAL-ASP-PRO-LYS-SER-LYS-ARG-THR-MET-GLN-
VAL-LEU-THR-THR-GLU-PRO-SER-VAL-GLN-MET-TYR-THR-
ALA-ASP-HIS-LEU-LEU-GLY-ASN-ILE-VAL-GLY-ALA-ASN-
GLY-VAL-LEU-TYR-ARG-GLN-ALA-ASP-ALA-LEU-ALA-LEU-
GLU-THR-GLN-HIS-PHE-PRO-ASP-SER-PRO-ASN-GLN-PRO-
THR-PHE-PRO-SER-THR-ARG-LEU-ASN-PRO-ASN-GLN-THR-
TYR-ASN-SER-VAL-THR-VAL-PHE-LYS-PHE-GLY-VAL-GLN-
LYS

5. Polypeptide ayant l'activité biologique de la mutarotase, caractérisé en ce qu'il comporte, entre les positions (A) et (B), la séquence polypeptidique

-ALA-THR-LEU-ASN-VAL-LYS-PRO-

6. Polypeptide ayant l'activité biologique de l'enzyme mutarotase, caractérisé en ce qu'il est codé par la séquence d'ADN selon la revendication 2 ou 3.

7. Séquence d'ADN selon l'une des revendications 1 à 3, caractérisée en ce que le gène structural provient du génome du genre *Acinetobacter*.

8. Séquence d'ADN selon l'une des revendications 1 à 3, caractérisée en ce que la séquence du répresseur de la tétracycline provient de *E. coli*.

9. Séquence d'ADN selon l'une des revendications 1 à 3, caractérisée en ce que la séquence promotrice provient de *E. coli.*

10. Séquence d'ADN selon la revendication 1, caractérisée en ce que le gène structural comporte la séquence nucléotidique comprise entre les positions (A) et (B) de la séquence d'ADN selon la revendication 2 ou 3, ou une séquence nucléotidique qui est apparentée à celle-ci par des variations voulues ou non voulues ou est hybridée avec elle.

11. Plasmide d'expression portant la désignation pWH1256 et le numéro de dépôt DSM 4228 P.

12. Organisme hôte, caractérisé en ce qu'il est transformé par au moins une molécule d'ADN selon l'une des revendications 1 à 3.

13. Organisme hôte selon la revendication 12, caractérisé en ce qu'il est *E. coli.*

14. Organisme hôte portant la désignation E. coli WH1256 et le numéro de dépôt DSM 4227.

15. Procédé pour la production d'un polypeptide ayant l'activité biologique de la mutarotase, par culture d'un micro-organisme dans un milieu de culture et isolement du polypeptide formé lors de l'expression, caractérisé en ce que, en tant que micro-organisme, on utilise un organisme hôte transformé par au moins une molécule d'ADN recombinant selon la revendication 1 ou 10.

16. Utilisation d'un polypeptide selon la revendication 4, 5 ou 6, pour l'accélération de réactions enzymatiques d'aldoses.
